Europäisches Patentamt

European Patent Office

Office européen des brevets

(11) Numéro de publication: **0 438 945 A1**

## (12) DEMANDE DE BREVET EUROPEEN

(21) Numéro de dépôt: 90403759.5

(22) Date de dépôt: 24.12.90

(51) Int. Cl.5: **G06F 15/20, A61B 5/0476**

(30) Priorité: 24.01.90 FR 9000796

(43) Date de publication de la demande:
**31.07.91 Bulletin 91/31**

(84) Etats contractants désignés:
**DE GB**

(71) Demandeur: **ETAT FRANCAIS représenté par le Ministre des Postes, Télécommunications et de l'Espace (CENTRE NATIONAL D'ETUDES DES TELECOMMUNICATIONS), 38-40 rue du Général Leclerc**
F-92131 Issy-les-Moulineaux(FR)

(72) Inventeur: **Blanchet, Gérard**
**48, rue Curie**
**F-94200 Ivry Sur Seine(FR)**
Inventeur: **Prado, Jacques**
**4, rue Saunier**
**F-91520 Egly(FR)**

(74) Mandataire: **Fort, Jacques**
**CABINET PLASSERAUD 84, rue d'Amsterdam**
**F-75009 Paris(FR)**

(54) Procédé et appareil de traitement de signal électro-encéphalographique.

(57) Le procédé de traitement de signal électro-encéphalographique permet de déterminer les stades du sommeil pendant la période de prélèvement du signal. On découpe le signal EEG en tranches temporelles de même durée ; on échantillonne numériquement chaque tranche du signal pour obtenir des blocs successifs de N échantillons ; pour chaque bloc i, on détermine M coefficients de corrélation normalisés $R_{i,k}$ , M étant très inférieur à N ; pour chaque bloc i, on calcule les distances D(i,j) entre les coefficients $R_{i,k}$ du bloc courant et les coefficients $R_{j,k}$ des centres de gravité des classes j déjà existantes ; on sélectionne le minimum D(i,p) des D(i,j) ; on affecte le bloc i à la classe p si D(i,p) est inférieur à un seuil déterminé, en remettant à jour le centre de gravité de la classe, tandis qu'on crée une nouvelle classe si D(i,p) est supérieur au seuil déterminé ; et on affecte chaque classe au stade le plus proche du point de vue répartition d'énergie entre bandes de fréquence.

FIG.1.

Xerox Copy Centre

## PROCÉDÉ ET APPAREIL DE TRAITEMENT DE SIGNAL ÉLECTRO-ENCÉPHALOGRAPHIQUE

L'invention concerne le traitement en temps réel d'un signal électro-encéphalographique, généralement dénommé EEG, en vue de la classification automatique des stades du sommeil d'un patient à partir des caractéristiques du signal EEG. Elle trouve des applications importantes, bien que non exclusives, dans l'étude du sommeil, effectuée dans les laboratoires hospitaliers, et dans l'étude des modifications du sommeil sous l'effet de médicaments, dans les laboratoires d'études des produits pharmaceutiques.

Les spécialistes de l'EEG reconnaissent en général six stades différents et les signaux obtenus pendant des tranches temporelles successives sont affectés à un des six stades.

A l'heure actuelle, la détermination des stades successifs du sommeil chez un patient s'effectue par dépouillement visuel d'un tracé du signal EEG et nécessite deux à trois jours de travail pour chaque tracé des signaux sur une nuit de sommeil, avec un taux de concordance entre résultats obtenus par différents opérateurs de l'ordre de 80%. Ce mode de dépouillement est pénible et très lent. L'opérateur doit, sur chaque tranche de signal, effectuer une analyse fréquentielle par observation d'un signal temporel, ce qui implique un lissage considérable et une appréciation subjective qui conduit à retenir pour la classification six stades seulement, communément dénommés veille, sommeil paradoxal, stade 1, stade 2, stade 3 et stade 4.

On a proposé un système comportant un micro-calculateur qui détermine les stades du sommeil par analyse des signaux provenant de plusieurs dérivations d'un électro-encéphalographe. Les signaux, échantillonnés à fréquence élevée (1000 Hz pour traiter des signaux ayant une fréquence maximale de 30 Hz) et numérisés, sont appliqués à un banc de filtres numériques ayant des fréquences caractéristiques fixées. Cet appareil transpose l'analyse visuelle et ne permet pas d'améliorer les performances de cette dernière.

L'invention vise à fournir un procédé et un appareil de traitement de signal EEG répondant mieux que ceux antérieurement connus aux exigences de la pratique, notamment en ce qu'ils permettent une classification totalement automatique de tranches temporelles successives du signal en temps réel, avec un taux de concordance avec la classification visuelle qui est du même ordre de grandeur qu'entre classifications visuelles effectuées par des opérateurs différents. Elle vise également à fournir un procédé permettant, le cas échéant, une analyse et une classification beaucoup plus fines que les procédés par observation directe.

Dans ce but, l'invention utilise une technique d'auto-apprentissage, ne faisant aucune hypothèse de départ sur les caractéristiques particulières du signal EEG traité.

De façon plus précise, l'invention propose un procédé de traitement en temps réel selon lequel : on découpe un signal EEG en tranches temporelles de même durée ; on échantillonne numériquement chaque tranche du signal pour obtenir des blocs successifs de N échantillons ; pour chaque bloc i, on détermine M coefficients de corrélation normalisés $R_{i,k}$ , M étant très inférieur à N ; pour chaque bloc i, on calcule les distances D(i,j) entre les coefficients $R_{i,k}$ du bloc courant et les coefficients $R_{j,k}$ des centres de gravité des classes j déjà existantes ; on sélectionne le minimum D(i,p) des D(i,j) et on affecte le bloc i à la classe p si D(i,p) est inférieur à un seuil déterminé, en remettant à jour le centre de gravité de la classe, tandis qu'on crée une nouvelle classe si D(i,p) est supérieur au seuil déterminé.

L'expérience a montré qu'il suffit d'une seule voie EEG, avantageusement la dérivation vertex-occipital, pour effectuer la classification, ce qui réduit la charge de calcul à un point tel qu'un même appareil à mini-calculateur peut analyser les signaux EEG provenant de plusieurs trajets simultanément.

Pratiquement, de bons résultats, conduisant à 80% de concordance avec les analyses visuelles, ont été obtenus avec échantillonnage à 100 Hz, N = 3000 (ce qui correspond à des tranches de 30 sec) et M = 16.

L'invention propose également un appareil permettant de mettre en oeuvre le procédé ci-dessus défini, comprenant :
- une voie d'acquisition ayant un filtre passe-bas ou passe-bande,
- un échantillonneur et un convertisseur analogique-numérique,
- un microprocesseur de prétraitement programmé pour calculer des coefficients de corrélation $R_{i,k}$ pourchaque tranche du signal EEG,
- un mini-calculateur de stockage des blocs de signaux et/ou des coefficients de corrélation, de création de classes de signaux, d'affectation des blocs et de remise à jour des classes.

La voie d'acquisition peut notamment avoir un filtre ayant une bande passante de 1 à 40 Hz (dans le cas d'un échantillonneur à 100 Hz), éventuellement un multiplexeur analogique permettant de traiter plusieurs signaux EEG, et un convertisseur analogique-numérique à 12 bits. Le microprocesseur chargé d'effectuer le prétraitement des données en temps réel peut alors être du type 16 bits et prévu pour transmettre les coefficients de corrélation vers le mini-calculateur par blocs de 150 octets à l'issue du prétraitement de chaque bloc. Le mini-calculateur peut être un ordinateur personnel traitant les données en temps réel et stockant les résultats définitifs, et éventuellement les résultats intermédiaires, en mémoire de masse.

L'invention sera mieux comprise à la lecture de la description qui suit d'un procédé constituant un mode particulier de mise en oeuvre de l'invention, et d'un appareil permettant de le mettre en oeuvre. La description se réfère aux dessins qui l'accompagnent, dans lesquels :

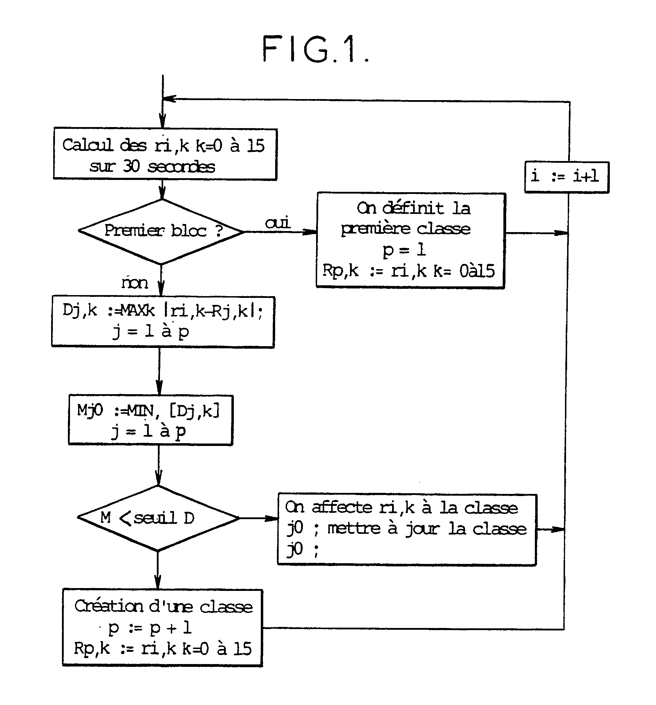

- la Figure 1 est un logigramme de principe des étapes de prétraitement et de classification du procédé ;
- la Figure 2 est un synoptique de principe montrant les composants essentiels mis en oeuvre dans l'appareil.

La partie du procédé mise en oeuvre en temps réel peut être considérée comme comportant :
- une étape d'acquisition des signaux EEG ;
- une étape de prétraitement ; et
- une étape de classification, qui peut être complétée par une analyse effectuée en temps différé.

L'acquisition implique la détection et l'amplification d'un signal EEG, avantageusement le signal vertex-occipital, et l'amplification analogique, jusqu'à un niveau permettant de réduire l'effet du bruit. Le signal amplifié est soumis à filtrage dans une bande considérée comme significative, habituellement de 1 à 40 Hz. Il est cependant possible d'utiliser une bande plus étroite, limitée par exemple à 30 Hz. Le signal est échantillonné à une fréquence au moins égale à deux fois la limite haute de la bande de filtrage, par exemple à 100 Hz. Les échantillons sont soumis à une conversion analogique-numérique, avec un nombre de niveaux de quantification suffisant pour obtenir la sélectivité désirée. Pratiquement, une quantification sur 12 bits, compatible avec l'utilisation d'un microprocesseur 16 bits, sera généralement satisfaisante.

Les échantillons sont soumis à l'étape de prétraitement et peuvent également être stockés sur une mémoire de masse permettant une exploitation et une analyse complémentaires en temps différé.

A cours du prétraitement on calcule, pour les blocs successifs de quelques milliers d'échantillons, plusieurs coefficients d'auto-corrélation du signal. Pratiquement, dans le cas d'une fréquence d'échantillonnage de 100 Hz, on peut utiliser des tranches de 30 sec, comportant N = 3000 échantillons et on peut caractériser chaque tranche d'ordre i par 16 coefficients de corrélation correspondant à des retards égaux au pas d'échantillonnage. On aura donc, pour la tranche d'ordre i, 16 coefficients :

$$R_{i,k} = (1/N) \sum_{n=1}^{N} S_n \, S_{n+k} \qquad (1)$$

où k varie de 0 à 15 pour chaque bloc de N échantillons.

La classification s'effectue de façon itérative, avec apprentissage au fur et à mesure du déroulement d'une expérience, dont la durée est en général de 8 à 10 heures. Le processus itératif permet de créer automatiquement des classes de répartition des blocs de signaux, d'y affecter les blocs et d'ajuster les classes. Il utilise un critère de ressemblance basé sur une distance de Tchebyshev, appliqué non pas aux coefficients $R_{i,k}$ de la formule (1), mais à des coefficients normalisés R* :

$$R^*_{i,k} = R_{i,k} / R_{i,0} \qquad (2)$$

Pour plus de simplicité, on désignera par la suite les coefficients normalisés par la simple notation R, et non pas R*. Le coefficient $R^*_{i,0}$ est toujours égal à l'unité, le coefficient $R_{i,0}$ est conservé et représente l'énergie de la tranche i de signal.

On utilise comme paramètre significatif, pour évaluer la ressemblance entre une tranche de signal et la tranche type d'une classe pré-existante, une distance constituée par le plus grand des quinze écarts entre un des coefficients de corrélation du signal de la tranche courante et le centre de gravité des coefficients correspondants des tranches déjà affectées aux classes pré-existantes.

La distance D(i,j), considérée comme significative pour apprécier la ressemblance entre la tranche d'ordre i du signal EEG et chaque classe j parmi les classes pré-existantes est :

$$D(i,j) = MAX_k |R_{i,k} - R_{j,k}| \qquad (3)$$

On détermine alors celui des coefficients D(i,j), dont le nombre est égal à celui des classes préexistantes, qui est le plus faible pour la tranche i de signal considéré. Cette valeur minimale

$$D(i,p) = Min_j D(i,j) \qquad (4)$$

révèle la classe pré-existante p pour laquelle il y a la plus grande ressemblance avec la tranche de signal d'ordre i.

Un test est alors effectué, par comparaison de D(i,p) avec un seuil D0 affichable par l'opérateur. Si D-(i,p) est inférieur au seuil, le bloc d'échantillons d'ordre i (ou la tranche de signal i) est considéré comme appartenant à la classe p et on remet simplement à jour le centre de gravité des coefficients de la classe p par l'opération

$$\begin{cases} R_{p,k} = [1/(N_p+1)] \ (N_p \ R_{p,k} + R_{i,k}) \\ \\ N_p = N_p + 1 \end{cases} \qquad (5)$$

Si, au contraire, D(i,p) est supérieur à D0, on crée une nouvelle classe dont le bloc courant constitue le premier bloc.

Ce seuil D0 est choisi en fonction du résultat d'expériences pour chaque appareillage particulier.

Il est suffisamment élevé pour que le nombre de classes généré au cours de l'ensemble de l'expérience ne soit pas excessif et disproportionné par rapport au nombre de stades du sommeil que permet de différencier l'analyse visuelle, ceci afin d'assurer une cohérence avec l'analyse visuelle. Il ne doit pas être trop faible, car le nombre de classes serait alors insuffisant. L'expérience permet de choisir la valeur la mieux appropriée. Une fois que cette valeur a été choisie pour un appareil donné, il n'est plus nécessaire de la modifier suivant le patient.

On voit que le procédé s'initialise seul : il suffit de fixer la valeur de seuil D. Le premier bloc constitue une première classe qu'on peut considérer comme la classe O et on mémorise alors les 16 coefficients de corrélation $R_{0,k}$ qui servent de termes de comparaison $R_{j,k}$ pour les coefficients $R_{1,k}$ du bloc suivant, d'ordre i = 1, qui est soit affecté à la classe pré-existante, soit utilisé pour créer une nouvelle classe et ainsi de suite.

A la fin de l'enregistrement, c'est-à-dire à l'issue de l'expérience, on dispose en mémoire :
- des centres de gravité des 15 coefficients $R_{j,k}$ (j ≠ 0) et
- de l'affectation de chacun des blocs d'échantillons à une classe.

On peut alors, par des processus mathématiques bien connus, établir pour chaque classe un modèle paramétrique auto-régressif, d'ordre 15 en cas de calcul de 16 coefficients de corrélation normalisés. On peut déduire de ce modèle un spectre de fréquence et calculer, pour plusieurs bandes de fréquence considérées comme particulièrement significatives pour l'affectation à un stade de sommeil, le pourcentage de l'énergie totale du signal qui est contenu dans la bande. L'expérience a montré qu'il était en général commode d'adopter trois bandes de fréquence allant respectivement de 1 à 4 Hz, de 4 à 7 Hz et de 12 à 14 Hz (cette répartition n'étant cependant pas la seule possible).

Si on désigne par $B_m$ le pourcentage d'énergie pour la bande d'ordre m (m prenant les valeurs 1, 2 et 3), on peut calculer, pour chaque classe, des coefficients $c_s$ en nombre égal à celui des stades de sommeil préalablement définis et qui sont en général ceux que l'analyse visuelle permet de différencier. Ces coefficients sont de la forme :

$$c_s = \sum_m \alpha_{m,s} \ B_m + \beta_s \qquad (6)$$

On affecte alors chacune des classes obtenues par l'analyse fréquentielle automatique à celui des stades de sommeil pour lequel la probabilité pour que le stade soit représentatif de la classe Cp est maximale. Cette probabilité P est donnée par :

$$P\ (C_p \in \text{stade } S) = \exp c_S / \sum_S \exp c_S \qquad (7)$$

Les coefficients $\alpha_{m,s}$ et $\beta_s$ peuvent être déterminés par analyse statistique sur un nombre d'expériences élevé, dépassant la centaine. Dans le cas envisagé plus haut de six stades de sommeil et de trois bandes de fréquence, les valeurs du Tableau I ont été trouvées satisfaisantes :

### Tableau I

| Bandes $B_m$ / Stades | $\alpha_{m,s}$ | | | $\beta_s$ |
|---|---|---|---|---|
| | 1 à 4 Hz | 4 à 7 Hz | 12 à 14 Hz | |
| Veille | 0,21 | 0,42 | 1,75 | − 14,16 |
| Sommeil paradoxal | 0,98 | 1,10 | 1,96 | − 33,92 |
| Stade 1 | 0,68 | 0,74 | 1,98 | − 22,83 |
| Stade 2 | 1,29 | 0,79 | 3,26 | − 40,90 |
| Stade 3 | 1,97 | 0,46 | 1,90 | − 54,84 |
| Stade 4 | 2,54 | 0,04 | 0,55 | − 74,30 |

Les valeurs de $\alpha_{m,s}$ peuvent varier, d'environ ± 5% dans la bande de 1 à 4 Hz, de ± 10% dans la bande de 4 à 7 Hz et de ± 15% dans la bande de 12 à 14 Hz. Ces possibilités de variation tiennent compte d'observations concernant le caractère plus ou moins significatif des trois bandes. Si on utilise uniquement la bande de 1 à 4 Hz, la classification est correcte dans 55% environ des cas. En y ajoutant l'utilisation de la bande 4 à 7 Hz, on améliore de 15% la classification. Enfin, l'utilisation de la bande de 12 à 14 Hz apporte encore une amélioration de 8% environ. Cette simple indication permet de constater que, dans certains cas, on pourra utiliser deux bandes seulement. Dans d'autres cas, où l'on recherche une analyse aussi fine que possible, on pourra adopter un nombre de bandes fréquentielles plus élevé.

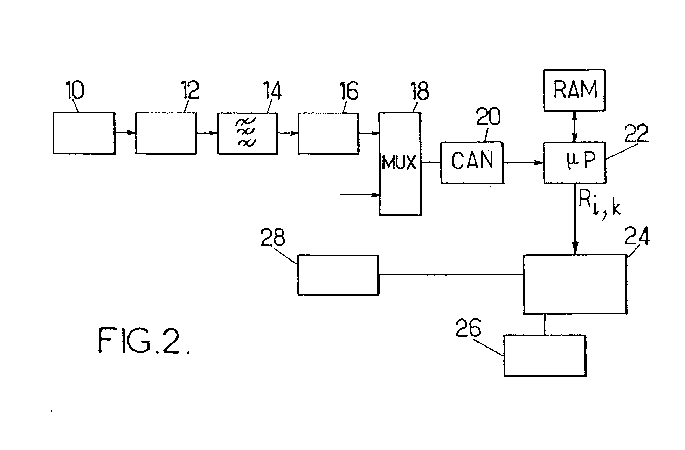

L'appareil de mise en oeuvre du procédé peut avoir la constitution de principe montrée en Figure 2. Le signal fourni par le capteur 10 et amplifié en 12, par exemple dans un banc d'enregistreurs graphiques de type classique, est appliqué à un filtre analogique passe-bande 14, dont la bande passante va de 1 à 40 Hz. Le signal de sortie du filtre 14 est appliqué à un échantillonneur 16 à 100 Hz qui attaque l'une des entrées d'un multiplexeur 18. Il est en effet possible de traiter simultanément plusieurs signaux EEG dans un même appareil, du fait du faible volume de calculs nécessaire. Les échantillons sont transformés en nombres de 12 bits par un convertisseur analogique-numérique 20 et appliqués à un microprocesseur 22 à mots de 16 bits, destiné à effectuer le prétraitement et à transmettre les résultats de ce prétraitement, constitués par les coefficients de corrélation, calculés sur chaque tranche de 30 sec, à un microcalculateur 24, généralement de type ordinateur personnel. La transmission peut s'effectuer par une liaison série asynchrone de type RS 232, par blocs de 150 octets à l'issue de chaque tranche de 30 sec. Ces 150 octets permettent de transférer également des informations supplémentaires, telles que chaque numéro de bloc, le nombre de saturations, les débordements éventuels de calcul.

Le logiciel de classification peut être implanté soit dans une mémoire associée au microprocesseur si celui-ci assume la fonction, soit en mémoire 26 du micro-calculateur 24 qui stocke, en mémoire de masse 28, toutes les données correspondant aux blocs successifs, telles que notamment les coefficients de corrélation R, un fichier d'enregistrement des blocs, un fichier de classes pour chaque patient, etc. Il calcule les coefficients c définis ci-dessus et les probabilités. Il affecte en conséquence les classes, qui se sont constituées par un processus d'auto-apprentissage, aux stades prédéfinis par l'expérimentateur, et donc répartit les tranches de signal entre les différents stades. Souvent, la valeur de seuil choisie est telle que le nombre de classes obtenues à la fin d'une nuit de sommeil va de 20 à 30, alors que le nombre de stades fixés par les spécialistes de l'EEG est de 6, ce nombre représentant le maximum pratique des stades entre lesquels il est possible d'effectuer une discrimination par observation visuelle du signal EEG. On peut en conséquence introduire des stades intermédiaires de sommeil, notés par exemple 1-1, 1-2,..., 2-3 qui sont utilisés chaque fois qu'aucune des probabilités calculées n'est supérieure à une valeur prédéterminée, par exemple de 70%.

Un logiciel d'analyse complémentaire peut être implanté en mémoire 26 du micro-calculateur 24. Ce logiciel permet de traiter les fichiers et d'effectuer des opérations supplémentaires, telles que la suppression de classes non significatives, l'association impérative d'une classe à un stade de sommeil, la visualisation graphique des résultats, des hypnogrammes résultant de critères de classification variés, etc. Une telle analyse permet à l'expérimentateur d'intervenir pour tenir compte de paramètres particuliers, tels que par exemple la tranche d'âge des sujets étudiés, pour tenir compte de la variation importante du signal EEG en fonction de l'âge.

L'invention est susceptible de nombreuses variantes de réalisation, en particulier en ce qui concerne l'appareil de mise en oeuvre. Les différentes fonctions peuvent être réparties de façon différente de celle décrite, aussi bien en ce qui concerne la répartition des traitements entre partie analogique et partie numérique que l'affectation des traitements numériques aux unités de calcul.

## Revendications

1. Procédé de traitement de signal électro-encéphalographique en vue de déterminer les stades du sommeil pendant la période de prélèvement du signal, **caractérisé en ce que :**

   (a) on découpe le signal EEG en tranches temporelles de même durée ;

   (b) on échantillonne numériquement chaque tranche du signal pour obtenir des blocs successifs de N échantillons ;

   (c) pour chaque bloc i, on détermine M coefficients de corrélation normalisés $R_{i,k}$, M étant très inférieur à M ;

   (d) pour chaque bloc i, on calcule les distances $D(i,j)$ entre les coefficients $R_{i,k}$ du bloc courant et les coefficients $R_{j,k}$ des centres de gravité des classes j déjà existantes ;

   (e) on sélectionne le minimum $D(i,p)$ des $D(i,j)$ ;

   (f) on affecte le bloc i à la classe p si $D(i,p)$ est inférieur à un seuil déterminé, en remettant à jour le centre de gravité de la classe, tandis qu'on crée une nouvelle classe si $D(i,p)$ est supérieur au seuil déterminé ; et

   (g) on affecte chaque classe au stade le plus proche du point de vue répartition d'énergie entre bandes de fréquence.

2. Procédé selon la revendication 1, caractérisé en ce que, au cours de l'étape (g), on établit pour chaque classe un modèle paramétrique auto-régressif; on en déduit un spectre de fréquence ; on calcule, pour plusieurs bandes de fréquence significatives pour l'affectation à un stade de sommeil, le pourcentage de l'énergie totale du signal qui est contenu dans la bande ; on calcule, pour chaque classe, des coefficients $c_s$ en nombre égal à celui des stades de sommeil préalablement définis ; et on affecte chacune des classes obtenues par l'analyse fréquentielle automatique à celui des stades de sommeil pour lequel la probabilité pour que le stade soit représentatif de la classe est maximale.

3. Procédé selon la revendication 2, caractérisé en ce que les trois bandes vont respectivement de 1 à 4 Hz, de 4 à 7 Hz et de 12 à 14 Hz.

4. Procédé selon la revendication 3, caractérisé en ce que lesdits coefficients sont de la forme

$$c_s = \sum_m \alpha_{m,s} \, B_m + \beta_s$$

où $\alpha$ et $\beta$ sont des coefficients différents suivant les stades du sommeil et la probabilité P est celle donnée par

$$P\ (C_p \in \text{stade } s) = \exp c_s / \sum_s \exp c_s$$

**5.** Procédé selon la revendication 4, caractérisé en ce que l'on adopte, pour les coefficients, les valeurs suivantes, correspondant à six stades de sommeil

| Bandes $B_m$ / Stades | $\alpha_{m,s}$ | | | $\beta_s$ |
|---|---|---|---|---|
| | 1 à 4 Hz | 4 à 7 Hz | 12 à 14 Hz | |
| Veille | 0,21 | 0,42 | 1,75 | − 14,16 |
| Sommeil paradoxal | 0,98 | 1,10 | 1,96 | − 33,92 |
| Stade 1 | 0,68 | 0,74 | 1,98 | − 22,83 |
| Stade 2 | 1,29 | 0,79 | 3,26 | − 40,90 |
| Stade 3 | 1,97 | 0,46 | 1,90 | − 54,84 |
| Stade 4 | 2,54 | 0,04 | 0,55 | − 74,30 |

**6.** Appareil de mise en oeuvre du procédé selon l'une quelconque des revendications précédentes, caractérisé en ce qu'il comprend :
- une voie d'acquisition ayant un filtre passe-bas ou passe-bande,
- un échantillonneur et un convertisseur analogique-numérique,
- un microprocesseur de prétraitement programmé pour calculer des coefficients de corrélation $R_{i,k}$ pourchaque tranche du signal EEG,
- un mini-calculateur de stockage des blocs de signaux et/ou des coefficients de corrélation, de création de classes de signaux, d'affectation des blocs et de remise à jour des classes.

**7.** Appareil selon la revendication 6, caractérisé en ce que la voie d'acquisition a un filtre (14) ayant une bande passante de 1 à 40 Hz et un échantillonneur de 100 Hz.

# FIG.1.

FIG.2.

**Office européen des brevets**

# RAPPORT DE RECHERCHE EUROPEENNE

Numéro de la demande

## EP 90 40 3759

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 306 346 (CNS, INC.)<br>* le document en entier *<br>— — — | 1 | G 06 F 15/20<br>A 61 B 5/0476 |
| A | US-A-4 214 591 (K. SATO ET AL)<br>* le document en entier *<br>— — — | 1 | |
| A | Proc of 8th annual conf of the IEEE/Engineering in Medicine and Biology Society, The Worthington Hotel-Fort Worth, Texas USA vol. 1, 07 novembre 1986, pages 418 - 422; A. Rosenfalck et al.: "On-line analysis of EEG"<br>* le document en entier *<br>— — — | 1 | |
| A | IEEE TRANSACTIONS ON BIO-MEDICAL ENGINEERING. vol. BME33, no. 10, octobre 1986, NEW YORK US pages 935 - 941; J.C. Principe et al.: "SAMICOS A Sleep Analyzing Microcomputer System"<br>* le document en entier *<br>— — — | 1 | |
| A | JOURNAL A. vol. 26, no. 2, avril 1985, ANTWERPEN BE pages 91 - 97; B. Lacroix et al.: "New algorithms for on-line automatic sleep scoring, and their application to mini and micro-computer"<br>* le document en entier *<br>— — — — — | 1 | **DOMAINES TECHNIQUES RECHERCHES (Int. Cl.5)**<br><br>G 06 F<br>A 61 B |

Le présent rapport de recherche a été établi pour toutes les revendications

| Lieu de la recherche | Date d'achèvement de la recherche | Examinateur |
|---|---|---|
| Berlin | 28 mars 91 | DURAND J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention

E : document de brevet antérieur, mais publié à la date de dépôt ou après cette date
D : cité dans la demande
L : cité pour d'autres raisons

---

& : membre de la même famille, document correspondant